(19) Europäisches Patentamt
European Patent Office
Office européen des brevets

(11) **EP 4 276 839 A1**

(12) **EUROPEAN PATENT APPLICATION**
published in accordance with Art. 153(4) EPC

(43) Date of publication:
**15.11.2023 Bulletin 2023/46**

(21) Application number: **21917615.3**

(22) Date of filing: **07.12.2021**

(51) International Patent Classification (IPC):
*G16C 20/40* (2019.01)

(52) Cooperative Patent Classification (CPC):
**G16C 20/40**

(86) International application number:
**PCT/JP2021/044992**

(87) International publication number:
**WO 2022/149394 (14.07.2022 Gazette 2022/28)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **07.01.2021 JP 2021001610**

(71) Applicant: **FUJIFILM Corporation
Tokyo 106-8620 (JP)**

(72) Inventors:
• **YARIMIZU, Hirokazu
Tokyo 106-8620 (JP)**
• **HIKIDA, Yasushi
Tokyo 106-8620 (JP)**

(74) Representative: **Meissner Bolte Partnerschaft
mbB
Widenmayerstrasse 47
80538 München (DE)**

(54) **INFORMATION PROCESSING DEVICE, INFORMATION PROCESSING METHOD, AND INFORMATION PROCESSING PROGRAM**

(57) An information processing apparatus includes at least one processor. The processor receives input of partial structure data indicating a partial structure of a chemical substance and a condition regarding an index value indicating performance of the chemical substance; extracts, for each of a plurality of known chemical substances, a known chemical substance including an input partial structure which is a partial structure indicated by the partial structure data and satisfying the condition from a database in which structure data indicating a structure of the chemical substance is recorded; extracts a partial structure other than the input partial structure, which is included in a structure of the extracted known chemical substance as a co-occurrence partial structure; and displays the extracted co-occurrence partial structure.

FIG. 5

Processed by Luminess, 75001 PARIS (FR)

**Description**

**BACKGROUND OF THE INVENTION**

1. Field of the Invention

The disclosed technology relates to an information processing apparatus, an information processing method, and an information processing program.

2. Description of the Related Art

The following technologies are known as technologies related to design support for chemical substances. For example, JP2019-040422A discloses a compound design apparatus comprising: partial structure selection means for selecting any partial structure of a compound; partial structure detection means for detecting a partial structure co-occurring with the partial structure selected by the partial structure selection means in compound data; and co-occurrence quantification means for quantifying a degree of co-occurrence for a plurality of the partial structures detected by the partial structure detection means.

**SUMMARY OF THE INVENTION**

A partial structure constituting a part of a chemical substance may have a great influence on performance of the chemical substance. Therefore, in designing a structure of a chemical substance exhibiting desired performance for the purpose of manufacturing, it is necessary to understand what partial structure the chemical substance should have. However, the performance of the chemical substance is changed not only by existence of the partial structure but also by a positional relationship and combination of the partial structure with other structures contained in the chemical substance. Since factors that affect the performance of the chemical substance are complicated, it is not easy to design a structure of the chemical substance after completely understanding such factors.

The disclosed technology has been made in view of the above points, and an object of the disclosed technology is to support a structural design of a chemical substance exhibiting desired performance.

An information processing apparatus according to an aspect of the disclosed technology comprises at least one processor, in which the processor receives input of partial structure data indicating a partial structure of a chemical substance and a condition regarding an index value indicating performance of the chemical substance; extracts, for each of a plurality of known chemical substances, a known chemical substance including an input partial structure which is a partial structure indicated by the partial structure data and satisfying the condition from a database in which structure data indicating a structure of the chemical substance is recorded; extracts a partial structure other than the input partial structure, which is included in a structure of the extracted known chemical substance as a co-occurrence partial structure; and displays the extracted co-occurrence partial structure.

The processor may display the extracted co-occurrence partial structure while showing a connection relationship with the input partial structure. The processor may calculate a co-occurrence probability which is a probability that the extracted co-occurrence partial structure appears together with the input partial structure. In a case where a plurality of the co-occurrence partial structures having different structures are extracted, the processor may display the extracted plurality of co-occurrence partial structures in order of the co-occurrence probability. The processor may display the extracted co-occurrence partial structure together with the co-occurrence probability calculated for the co-occurrence partial structure.

An information processing method according to another aspect of the disclosed technology is a method in which a processor of an information processing apparatus executes a process comprising: receiving input of partial structure data indicating a partial structure of a chemical substance and a condition regarding an index value indicating performance of the chemical substance; extracting, for each of a plurality of known chemical substances, a known chemical substance including an input partial structure which is a partial structure indicated by the partial structure data and satisfying the condition from a database in which structure data indicating a structure of the chemical substance is recorded; extracting a partial structure other than the input partial structure, which is included in a structure of the extracted known chemical substance as a co-occurrence partial structure; and displaying the extracted co-occurrence partial structure.

An information processing program according to still another aspect of the disclosed technology is a program for causing a processor of an information processing apparatus to execute a process comprising: receiving input of partial structure data indicating a partial structure of a chemical substance and a condition regarding an index value indicating performance of the chemical substance; extracting, for each of a plurality of known chemical substances, a known chemical substance including an input partial structure which is a partial structure indicated by the partial structure data and satisfying the condition from a database in which structure data indicating a structure of the chemical substance is recorded; extracting a partial structure other than the input partial structure, which is included in a structure of the extracted known chemical substance as a co-occurrence partial structure; and displaying the extracted co-occurrence partial structure.

According to the disclosed technology, it is possible to support a structural design of a chemical substance exhibiting desired performance.

## BRIEF DESCRIPTION OF THE DRAWINGS

Fig. 1 is a diagram showing an example of a hardware configuration of an information processing apparatus according to an embodiment of the disclosed technology.

Fig. 2 is a diagram showing an example of structure data of a chemical substance represented in a graph format.

Fig. 3 is a diagram showing an example of a chemical substance database according to the embodiment of the disclosed technology.

Fig. 4 is a diagram showing an example of a partial structure database according to the embodiment of the disclosed technology.

Fig. 5 is a functional block diagram showing an example of a functional configuration of the information processing apparatus according to the embodiment of the disclosed technology.

Fig. 6 is a diagram showing an example of an input partial structure according to the embodiment of the disclosed technology.

Fig. 7 is a diagram showing an example of a display form of a co-occurrence partial structure according to the embodiment of the disclosed technology.

Fig. 8 is a flowchart showing an example of a flow of display processing according to the embodiment of the disclosed technology.

Fig. 9 is a diagram showing a structure of a carboxylic acid.

Fig. 10 is a diagram showing a structure of a maleic acid.

Fig. 11 is a diagram showing a structure of a carboxyl group which is an example of an input partial structure.

## DESCRIPTION OF THE PREFERRED EMBODIMENTS

Hereinafter, an example of an embodiment of the disclosed technology will be described with reference to the drawings. In each drawing, the same or equivalent components and parts are designated by the same references, and redundant descriptions will not be repeated as appropriate.

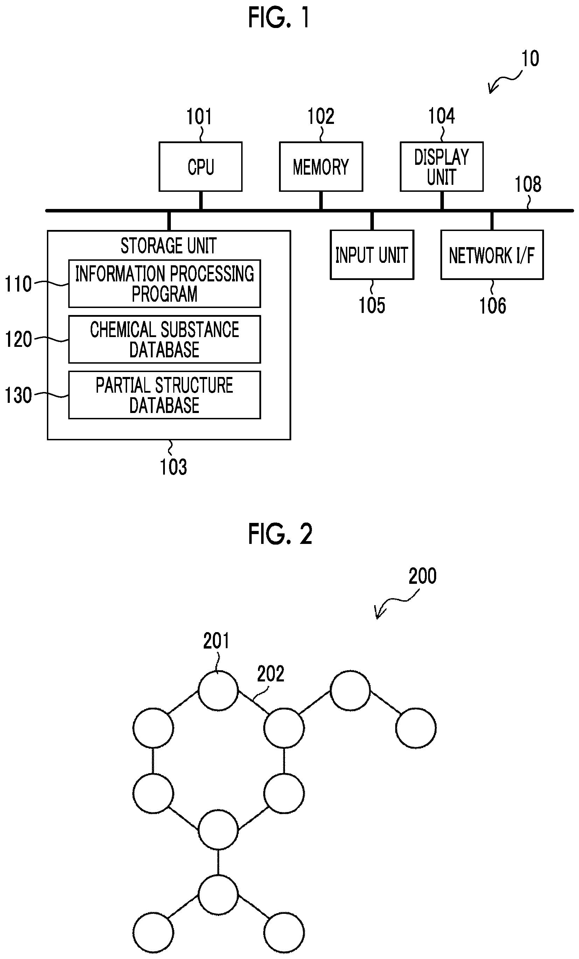

Fig. 1 is a diagram showing an example of a hardware configuration of an information processing apparatus 10 according to an embodiment of the disclosed technology. The information processing apparatus 10 includes a central processing unit (CPU) 101, a memory 102 as a temporary storage area, and a storage unit 103. In addition, the information processing apparatus 10 includes a display unit 104 such as a liquid crystal display, an input unit 105 including an input device such as a keyboard and a mouse, and a network interface (I/F) 106 connected to a network. The CPU 101, the memory 102, the storage unit 103, the display unit 104, the input unit 105, and the network I/F 106 are each connected to a bus 108.

The storage unit 103 is realized by, for example, a nonvolatile storage medium such as a hard disk drive (HDD), a solid state drive (SSD), or a flash memory. An information processing program 110, a chemical substance database 120, and a partial structure database 130 are stored in the storage unit 103. The CPU 101 reads out the information processing program 110 from the storage unit 103, then loads the information processing program 110 into the memory 102, and executes the information processing program. An example of the information processing apparatus 10 is a server computer or the like. The CPU 101 is an example of a processor in the disclosed technology.

The information processing apparatus 10 is used for a structural design of a chemical substance and has a function as a molecular design editor. Structure data representing a structure of a chemical substance handled by the information processing apparatus 10 according to the present embodiment is represented in a graph format. Fig. 2 is a diagram showing an example of structure data 200 of a chemical substance represented in a graph format. In the structure data 200 represented in a graph format, atoms constituting the chemical substance are represented by nodes 201, and bonds between the atoms are represented by edges 202. The format of the structure data handled by the information processing apparatus 10 is not limited to the graph format and may be, for example, a character string format such as a deoxyribonucleic acid (DNA) base sequence.

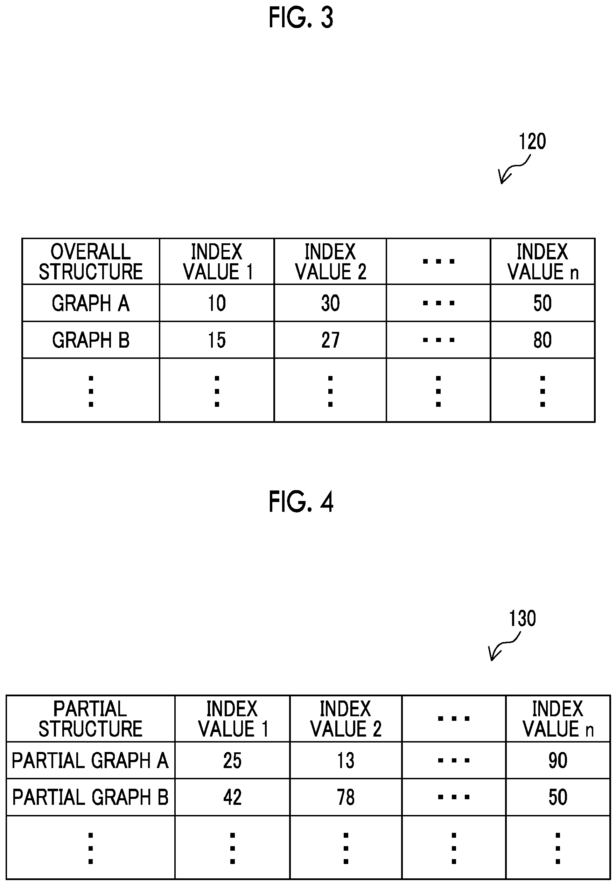

Fig. 3 is a diagram showing an example of the chemical substance database 120 stored in the storage unit 103. The chemical substance database 120 has recorded therein structure data representing an overall structure of the chemical substance for each of a plurality of known chemical substances. The structure data is represented in a graph format. At least one index value representing the performance of the chemical substance is associated with each piece of the structure data. Examples of the index value include a boiling point, a melting point, a glass transition temperature, a partition coefficient, a density, a viscosity, a thermal expansion factor, and a molecular weight. The index value may be, for example, an actually measured value obtained by a past experiment or a nominal value.

Fig. 4 is a diagram showing an example of the partial structure database 130 stored in the storage unit 103. The partial structure database 130 has recorded therein partial structure data representing the partial structure for each of a plurality of known partial structures. The partial structure is a partial structure of a structure, which constitutes a chemical substance. Examples of the partial structure include a structure of a functional group such as a carboxyl group, an aldehyde group, and a hydroxyl group. The structure data of the partial structure is represented in a graph format. At least one index value representing performance of the partial structure is associated with each piece of the structure data of the partial structure. Examples of the index value include presence or absence of carcinogenicity, presence or absence of toxicity, and a degree indicating a solubility in water. The index value may be, for example, an actually measured value obtained by a past experiment or a nominal value.

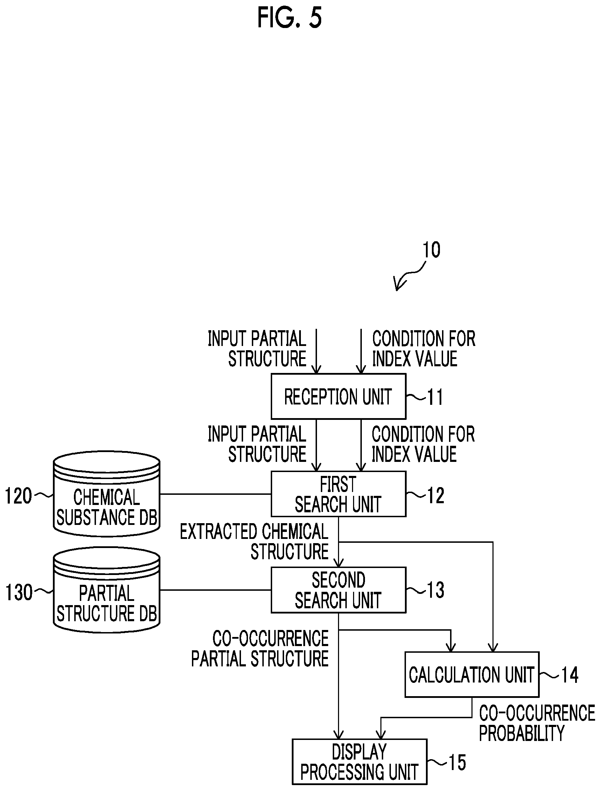

Fig. 5 is a functional block diagram showing an example of a functional configuration of the information processing apparatus 10. The information processing apparatus 10 includes a reception unit 11, a first search unit 12, a second search unit 13, a calculation unit 14, and a display processing unit 15. By executing the information processing program 110 by the CPU 101, the information processing apparatus 10 functions as the reception unit 11, the first search unit 12, the second search unit 13, the calculation unit 14, and the display processing unit 15.

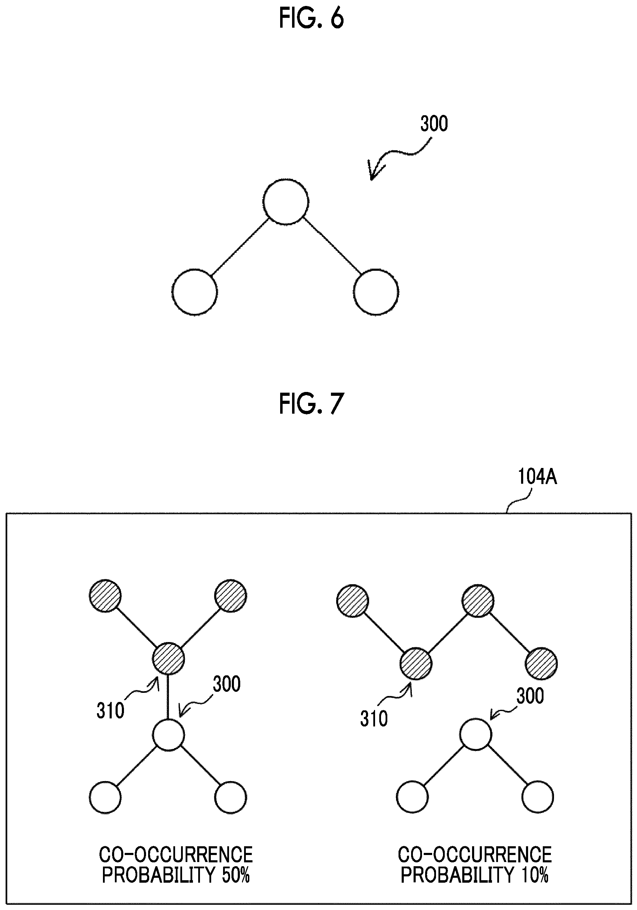

A user who performs a structural design of a chemical substance using the information processing apparatus 10 inputs to the information processing apparatus 10 a partial structure that can be included in the chemical substance to be designed. The partial structure input to the information processing apparatus 10 is hereinafter referred to as an input partial structure. The input partial structure can be input to the information processing apparatus 10 by operating the input unit 105. The reception unit 11 receives partial structure data indicating the input partial structure input by the user. Fig. 6 is a diagram showing an example of an input partial structure 300.

In addition, the user establishes a condition regarding a specific index value based on performance to be provided in the chemical substance to be designed and inputs the established condition to the information processing apparatus 10. For example, in a case of designing a structure of a chemical substance having a boiling point of 150°C or lower, information indicating "boiling point of 150°C or lower" is input as the condition regarding the index value. The condition regarding the index value can be input to the information processing apparatus 10 by operating the input unit 105. The reception unit 11 receives the condition regarding the specific index value input by the user. There may be two or more conditions regarding the specific index value. The reception unit 11 supplies the received input partial structure and condition regarding the specific index value to the first search unit 12.

The first search unit 12 searches for and extracts a chemical substance including the input partial structure and satisfying the condition regarding the index value from the chemical substance database 120. That is, the first search unit 12 extracts a chemical substance that includes the input partial structure and can exhibit the required performance from a plurality of known chemical substances included in the chemical substance database 120. In a case where a plurality of chemical substances including the input partial structure and satisfying the condition regarding the index value are present in the chemical substance database 120, the first search unit 12 extracts all the corresponding chemical substances. The first search unit 12 supplies structure data indicating a structure of the extracted chemical substance to the second search unit 13 and the calculation unit 14. The structure of the chemical substance extracted by the first search unit 12 will be referred to hereinafter as an extracted chemical structure.

The second search unit 13 extracts a partial structure other than the input partial structure, which is included in the extracted chemical structure, as a co-occurrence partial structure. That is, the co-occurrence partial structure is a partial structure that exists together with the input partial structure in a certain chemical substance. The second search unit 13 extracts the co-occurrence partial structure by referring to the partial structure database 130. That is, in a case where the second search unit 13 finds, in the extracted chemical structure, a partial structure other than the input partial structure, which matches the partial structure recorded in the partial structure database 130, the second search unit 13 extracts the partial structure as the co-occurrence partial structure. In a case where a plurality of the corresponding partial structures described above are found in the extracted chemical structure, the second search unit 13 extracts all the corresponding partial structures as the co-occurrence partial structures. The second search unit 13 supplies the partial structure data indicating the co-occurrence partial structure to the display processing unit 15 and the calculation unit 14.

The calculation unit 14 calculates a co-occurrence probability which is a probability that the co-occurrence partial structure appears together with the input partial structure. In a case where a plurality of co-occurrence partial structures having different structures are extracted, the calculation unit 14 calculates the co-occurrence probability for each of the plurality of co-occurrence partial structures. The co-occurrence probability may be expressed by, for example, the following Equation (1). In Equation (1), P is the co-occurrence probability, A is the number of extractions of one kind of co-occurrence partial structure extracted by the second search unit 13, and B is the number of extractions of the chemical substance extracted by the first search unit 12. In addition, B may be the total number of chemical substances recorded in the chemical substance database 120. It is considered that a co-occurrence partial structure having a relatively high co-occurrence probability tends to have a relatively high possibility of satisfying the condition regarding the index value by existing together with the input partial structure.

$$P = A/B \times 100 \ [\%] \ ... \ (1)$$

The display processing unit 15 performs a process of displaying the co-occurrence partial structure extracted by the second search unit 13 on the display unit 104. Fig. 7 is a diagram showing an example of a display form of a co-occurrence partial structure 310 displayed on a display screen 104A of the display unit 104.

The display processing unit 15 performs a process of displaying the extracted co-occurrence partial structure 310 while showing a connection relationship with the input partial structure 300. The co-occurrence partial structure 310 and the input partial structure 300 are displayed in a graph format. For example, in a case where the co-occurrence partial structure 310 and the input partial structure 300 are directly connected, a connection location is represented by an edge. The display processing unit 15 may perform a process of displaying the co-occurrence partial structure 310 and the input partial structure 300 in an identifiable manner. For example, a process may be performed in which a node constituting the co-occurrence partial structure 310 and a node constituting the input partial structure 300 are displayed in different colors. The display processing unit 15 may perform a process of displaying only the co-occurrence partial structure 310 out of the co-occurrence partial structure 310 and the input partial structure 300. In addition, the display processing unit 15 may perform a process of displaying an overall structure including both the input partial structure 300 and the co-occurrence partial structure 310, that is, an overall structure of the chemical substance extracted by the first search unit 12.

In a case where a plurality of co-occurrence partial structures having different structures are extracted, the display processing unit 15 performs a process of arranging and displaying the extracted plurality of co-occurrence partial structures in order of the co-occurrence probability calculated by the calculation unit 14. For example, as shown in Fig. 7, the display processing unit 15 may perform a process of arranging and displaying the extracted plurality of co-occurrence partial structures 310 in order from the left to the right of the display screen 104A in descending order of the co-occurrence probability. In addition, the extracted plurality of co-occurrence partial structures 310 may be arranged and displayed in order from the top to the bottom of the display screen 104A in descending order of the co-occurrence probability. The display processing unit 15 may perform a process of displaying the co-occurrence partial structure together with the co-occurrence probability.

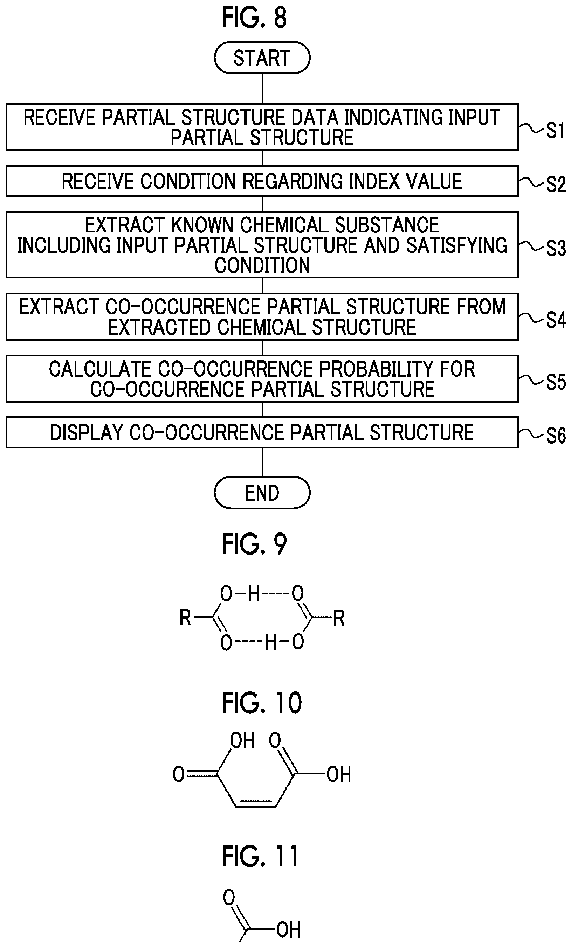

Fig. 8 is a flowchart showing an example of a flow of display processing implemented by executing the information processing program 110 by the CPU 101.

In step S1, the reception unit 11 receives partial structure data indicating the input partial structure input by the user by operating the input unit 105. In step S2, the reception unit 11 receives the condition regarding the specific index value input by the user by operating the input unit 105. The condition regarding the specific index value is established based on performance that the chemical substance to be designed should have.

In step S3, the first search unit 12 searches for and extracts from the chemical substance database 120 a known chemical substance including the input partial structure received in step S1 and satisfying the condition regarding the index value received in step S2.

In step S4, the second search unit 13 refers to the partial structure database 130 to extract a partial structure other than the input partial structure, which is included in the structure (that is, the extracted chemical structure) of the known chemical substance extracted in step S3, as the co-occurrence partial structure.

In step S5, the calculation unit 14 calculates a co-occurrence probability which is a probability that the co-occurrence partial structure extracted in step S4 appears together with the input partial structure. The calculation unit 14 calculates the co-occurrence probability based on, for example, the above Equation (1).

In step S6, the display processing unit 15 performs a process of displaying the co-occurrence partial structure extracted in step S4 on the display unit 104. Specifically, the display processing unit 15 performs a process of displaying the co-occurrence partial structure while showing a connection relationship with the input partial structure. In a case where a plurality of co-occurrence partial structures having different structures are extracted, the display processing unit 15 performs a process of arranging and displaying the extracted plurality of co-occurrence partial structures in order of the co-occurrence probability.

A partial structure constituting a part of a chemical substance may have a great influence on performance of the chemical substance. Therefore, in designing a chemical substance exhibiting desired performance, it is necessary to understand what partial structure the chemical substance should have.

Here, a carboxylic acid shown in Fig. 9 generally forms a dimer by hydrogen bonding. A chemical substance that forms a hydrogen bond, such as a carboxylic acid, has a higher boiling point than a chemical substance having substantially the same molecular weight that does not form a hydrogen bond. On the other hand, a maleic acid shown in Fig. 10 is one of carboxylic acids but does not form a hydrogen bond with another molecule because two carboxyl groups are present adjacent to each other. Therefore, the boiling point of the maleic acid is relatively low even though it is a carboxylic acid.

As described above, the performance (for example, the boiling point) of the chemical substance changes not only by the existence of the partial structure but also by a positional relationship and combination of the partial structure with other structures contained in the chemical substance. Since factors that affect the performance of the chemical substance are complicated, it is not easy to design a structure of the chemical substance after completely understanding such factors.

With the information processing apparatus 10 according to the embodiment of the disclosed technology, a known chemical substance including an input partial structure and satisfying a condition (that is, a requirement regarding performance) regarding a specific index value is extracted, and a partial structure other than the input partial structure, which is included in the structure the extracted chemical substance is extracted and displayed as a co-occurrence partial structure.

For example, assume a case where a carboxyl group shown in Fig. 11 is input to the information processing apparatus 10 as an input partial structure, and "a boiling point of 150°C or lower" is input as a condition regarding a specific index value. In this case, the maleic acid shown in Fig. 10 is extracted as a known chemical substance that includes an input partial structure and satisfies the condition. Then, of two carboxyl groups contained in the maleic acid, a carboxyl group different from the input partial structure is extracted and displayed as the co-occurrence partial structure.

As described above, according to the information processing apparatus 10, a partial structure that may satisfy the condition regarding the index value in a case of existing together with the input partial structure is presented to the user as the co-occurrence partial structure. The user can adopt the presented co-occurrence partial structure as a candidate for the partial structure to be included in the chemical substance to be designed. As described above, with the information processing apparatus 10 according to the embodiment of the disclosed technology, it is possible to support the structural design of the chemical substance exhibiting the desired performance.

In addition, according to the information processing apparatus 10, since the co-occurrence partial structure extracted from the structure of the known chemical substance extracted from the chemical substance database 120 is displayed, a possibility that the displayed co-occurrence partial structure is useful for exhibiting a desired performance can be increased. In addition, according to the information processing apparatus 10, since the extracted co-occurrence partial structure is displayed in a state where a connection relationship with the input partial structure is shown, the design support can be performed more effectively. In addition, according to the information processing apparatus 10, in a case where a plurality of co-occurrence partial structures having different structures are extracted, since the extracted plurality of co-occurrence partial structures are displayed in a state of being arranged in order of the co-occurrence probability, it is possible to specify a co-occurrence partial structure with a relatively high possibility of satisfying the condition regarding the index value.

In the present embodiment, a case where a partial structure that matches the partial structure recorded in the partial structure database 130 among partial structures included in the extracted chemical structure is extracted as the co-occurrence partial structure is illustrated, but the disclosed technology is not limited to this aspect. A partial structure selected randomly or according to a predetermined rule from the extracted chemical structure may be extracted as the co-occurrence partial structure.

In the above-described embodiment, for example, as a hardware structure of a processing unit that executes various types of processing such as the reception unit 11, the first search unit 12, the second search unit 13, the calculation unit 14, and the display processing unit 15, various types of processors shown below can be used. As described above, in addition to the CPU which is a general-purpose processor executing software (program) to function as various types of processing units, the various types of processors include a programmable logic device (PLD) which is a processor capable of changing a circuit configuration after manufacture such as a field programmable gate array (FPGA), a dedicated electric circuitry which is a processor having a circuit configuration exclusively designed to execute specific processing such as an application specific integrated circuit (ASIC), and the like.

One processing unit may be configured of one of the various types of processors, or a combination of two or more processors of the same type or different types (for example, a combination of a plurality of FPGAs, or a combination of a CPU and an FPGA). In addition, a plurality of processing units may be configured by one processor.

As an example of configuring a plurality of processing units with one processor, first, there is a form in which, as typified by computers such as a client and a server, one processor is configured by combining one or more CPUs and software, and the processor functions as a plurality of processing units. Second, as typified by a system on chip (SoC) or the like, there is a form in which a processor that realizes functions of an entire system including a plurality of processing units with one integrated circuit (IC) chip is used. As described above, the various types of processing units are configured using one or more of the various types of processors as a hardware structure.

Furthermore, as the hardware structure of the various types of processors, more specifically, an electric circuitry in which circuit elements such as semiconductor elements are combined can be used.

Further, in the above-described embodiment, an aspect in which the information processing program 110 is stored (installed) in advance in the storage unit 103, but the disclosed technology is not limited thereto. The information processing program 110 may be provided in a form recorded in a recording medium such as a compact disc read only memory (CD-ROM), a digital versatile disc read only memory (DVD-ROM), and a universal serial bus (USB) memory. Further, the information processing program 110 may be downloaded from an external device via a network.

The disclosure of JP 2021-001610 filed on January 7, 2021 is incorporated herein by reference in its entirety. In addition, all publications, patent applications, and technical standards described in this specification are incorporated by reference herein to the same extent as in a case where it is specifically and individually stated that individual documents, patent applications, and technical standards are incorporated by reference.

**Claims**

1. An information processing apparatus comprising at least one processor,
   wherein the processor is configured to:

   receive input of partial structure data indicating a partial structure of a chemical substance and a condition regarding an index value indicating performance of the chemical substance;
   extract, for each of a plurality of known chemical substances, a known chemical substance including an input partial structure which is a partial structure indicated by the partial structure data and satisfying the condition from a database in which structure data indicating a structure of the chemical substance is recorded;
   extract a partial structure other than the input partial structure, which is included in a structure of the extracted known chemical substance as a co-occurrence partial structure; and
   display the extracted co-occurrence partial structure.

2. The information processing apparatus according to claim 1, wherein the processor is configured to display the extracted co-occurrence partial structure while showing a connection relationship with the input partial structure.

3. The information processing apparatus according to claim 1 or 2, wherein the processor is configured to:

   calculate a co-occurrence probability which is a probability that the extracted co-occurrence partial structure appears together with the input partial structure; and
   in a case where a plurality of the co-occurrence partial structures having different structures are extracted, display the extracted plurality of co-occurrence partial structures in order of the co-occurrence probability.

4. The information processing apparatus according to claim 3, wherein the processor is configured to display the extracted co-occurrence partial structure together with the co-occurrence probability calculated for the co-occurrence partial structure.

5. An information processing method in which a processor of an information processing apparatus executes a process comprising:

   receiving input of partial structure data indicating a partial structure of a chemical substance and a condition regarding an index value indicating performance of the chemical substance;
   extracting, for each of a plurality of known chemical substances, a known chemical substance including an input partial structure which is a partial structure indicated by the partial structure data and satisfying the condition from a database in which structure data indicating a structure of the chemical substance is recorded;
   extracting a partial structure other than the input partial structure, which is included in a structure of the extracted known chemical substance as a co-occurrence partial structure; and
   displaying the extracted co-occurrence partial structure.

6. An information processing program for causing a processor of an information processing apparatus to execute a process comprising:

   receiving input of partial structure data indicating a partial structure of a chemical substance and a condition regarding an index value indicating performance of the chemical substance;
   extracting, for each of a plurality of known chemical substances, a known chemical substance including an input partial structure which is a partial structure indicated by the partial structure data and satisfying the condition from a database in which structure data indicating a structure of the chemical substance is recorded;
   extracting a partial structure other than the input partial structure, which is included in a structure of the extracted known chemical substance as a co-occurrence partial structure; and
   displaying the extracted co-occurrence partial structure.

# FIG. 1

10

101  102  104

| CPU | MEMORY | DISPLAY UNIT |
|---|---|---|

108

STORAGE UNIT

110 — INFORMATION PROCESSING PROGRAM

120 — CHEMICAL SUBSTANCE DATABASE

130 — PARTIAL STRUCTURE DATABASE

INPUT UNIT

NETWORK I/F

105    106

103

# FIG. 2

200

201

202

## FIG. 3

120

| OVERALL STRUCTURE | INDEX VALUE 1 | INDEX VALUE 2 | · · · | INDEX VALUE n |
|---|---|---|---|---|
| GRAPH A | 10 | 30 | · · · | 50 |
| GRAPH B | 15 | 27 | · · · | 80 |
| ⋮ | ⋮ | ⋮ | ⋮ | ⋮ |

## FIG. 4

130

| PARTIAL STRUCTURE | INDEX VALUE 1 | INDEX VALUE 2 | · · · | INDEX VALUE n |
|---|---|---|---|---|
| PARTIAL GRAPH A | 25 | 13 | · · · | 90 |
| PARTIAL GRAPH B | 42 | 78 | · · · | 50 |
| ⋮ | ⋮ | ⋮ | ⋮ | ⋮ |

# FIG. 5

10

INPUT PARTIAL STRUCTURE    CONDITION FOR INDEX VALUE

RECEPTION UNIT ~11

INPUT PARTIAL STRUCTURE    CONDITION FOR INDEX VALUE

120~ CHEMICAL SUBSTANCE DB

FIRST SEARCH UNIT ~12

EXTRACTED CHEMICAL STRUCTURE

130~ PARTIAL STRUCTURE DB

SECOND SEARCH UNIT ~13

CO-OCCURRENCE PARTIAL STRUCTURE

CALCULATION UNIT ~14

CO-OCCURRENCE PROBABILITY

DISPLAY PROCESSING UNIT ~15

# FIG. 6

300

# FIG. 7

104A

310    300

310    300

CO-OCCURRENCE
PROBABILITY 50%

CO-OCCURRENCE
PROBABILITY 10%

## FIG. 8

START

RECEIVE PARTIAL STRUCTURE DATA INDICATING INPUT PARTIAL STRUCTURE ~S1

RECEIVE CONDITION REGARDING INDEX VALUE ~S2

EXTRACT KNOWN CHEMICAL SUBSTANCE INCLUDING INPUT PARTIAL STRUCTURE AND SATISFYING CONDITION ~S3

EXTRACT CO-OCCURRENCE PARTIAL STRUCTURE FROM EXTRACTED CHEMICAL STRUCTURE ~S4

CALCULATE CO-OCCURRENCE PROBABILITY FOR CO-OCCURRENCE PARTIAL STRUCTURE ~S5

DISPLAY CO-OCCURRENCE PARTIAL STRUCTURE ~S6

END

## FIG. 9

## FIG. 10

## FIG. 11

## INTERNATIONAL SEARCH REPORT

| International application No. |
| --- |
| **PCT/JP2021/044992** |

**A. CLASSIFICATION OF SUBJECT MATTER**

*G16C 20/40*(2019.01)i
FI: G16C20/40

According to International Patent Classification (IPC) or to both national classification and IPC

**B. FIELDS SEARCHED**

Minimum documentation searched (classification system followed by classification symbols)

G16C20/40

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | JP 2019-40422 A (THE NATIONAL INSTITUTE OF ADVANCED INDUSTRIAL SCIENCE AND TECHNOLOGY) 14 March 2019 (2019-03-14) entire text, all drawings | 1-6 |
| A | JP 2020-194488 A (FUJITSU LTD.) 03 December 2020 (2020-12-03) entire text, all drawings | 1-6 |
| A | JP 2007-277188 A (HITACHI, LTD.) 25 October 2007 (2007-10-25) entire text, all drawings | 1-6 |

☐ Further documents are listed in the continuation of Box C.   ☑ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **17 February 2022** | **01 March 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**
**Information on patent family members**

International application No.

**PCT/JP2021/044992**

| Patent document cited in search report | | | Publication date (day/month/year) | Patent family member(s) | Publication date (day/month/year) |
|---|---|---|---|---|---|
| JP | 2019-40422 | A | 14 March 2019 | (Family: none) | |
| JP | 2020-194488 | A | 03 December 2020 | US 2020/0381085 A1 entire text, all drawings | |
| JP | 2007-277188 | A | 25 October 2007 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019040422 A
- JP 2021001610 A